# EUROPÄISCHE PATENTANMELDUNG

(11) **EP 3 777 782 A1**
(43) Veröffentlichungstag der Anmeldung: **17.02.2021**
(21) Anmeldenummer: 19191683.2
(22) Anmeldetag: 14.08.2019
(51) Int. Cl.: A61F 2/958, A61B 5/107, A61B 17/12, A61M 25/10

(54) **LERNENDES MEDIZINISCHES SYSTEM FÜR EINE OPTIMALE STENTEINBETTUNG BEI DER ANGIOPLASTIE**

(71) Anmelder: BIOTRONIK AG, 8180 Bülach (CH)
(72) Erfinder: Wesselmann, Matthias, 8455 Rüdlingen (CH)
(74) Vertreter: Galander, Marcus

(57) **Zusammenfassung**

Die Erfindung betrifft ein medizinisches System (1) zur Unterstützung der Einbettung eines Stents (2) in ein Gefäß eines Patienten im Bereich einer Stenose während einer Angioplastie, mit: einem Ballonkatheter (3), der einen in einer axialen Richtung (A) erstreckten Ballon (4) aufweist und optional einen darauf angeordneten Stent (2), wobei der Ballon (4) zum Aufweiten des Stents (2) in radialer Richtung (R) dehnbar ist, und wobei das medizinische System (1) zumindest einen Sensor (5) aufweist, der dazu ausgebildet ist, einen Istwert eines Ballonparameters (D) zu messen; einer Datenverarbeitungseinheit (6), die dazu ausgebildet ist, folgende Daten (W) zu erfassen: Patientendaten, Daten zur Angioplastie, Daten zur aktuellen Situation des Patienten, Stenosedaten, einen Ballonparameter; einer Datenbankeinheit (7), wobei die Datenverarbeitungseinheit (6) dazu ausgebildet ist, die Daten an die Datenbankeinheit (7) zu übermitteln, wobei die Datenbankeinheit (7) dazu ausgebildet ist, die Daten in einer Datenbank (70) zu Speichern und zu analysieren, und wobei die Datenbankeinheit (7) dazu ausgebildet ist, eine multivariate Analyse der besagten Daten durchzuführen um eine Eintrittswahrscheinlichkeit einer Komplikation bei der Angioplastie abzuschätzen und/oder eine Handlungsempfehlung (V) für eine die Angioplastie durchführende Person zu erzeugen und der Person über eine Benutzerschnittstelle (9) des medizinischen Systems (1) anzuzeigen.

## Beschreibung

Die vorliegende Erfindung betrifft ein medizinisches System zur Unterstützung der Implantation eines vaskulären Implantats, insbesondere der Einbettung eines Stents in ein Gefäß eines Patienten im Bereich einer Stenose während einer Angioplastie.

Beim Setzen eines Stents im Rahmen einer Angioplastie zur Aufweitung einer Stenose eines Gefäßes steht der behandelnden Arzt aktuell vor der großen Herausforderung den optimalen Aufweitdurchmesser des zu setzenden Stents zu bestimmen und entsprechend einzustellen. Wird der Stent zu sehr aufgeweitet, droht eine Ruptur der angrenzenden Gefäßwandung. Wird der Stent hingegen nicht in ausreichender Weise aufgeweitet, kann es zu späten Stentthrombosen (im implantierten Stent bildet sich teilweise Monate bis Jahre später erneut eine Thrombose) kommen, die eine erhebliche Komplikation darstellen.

Rupturen bedeuten für den Arzt immer eine starke Stresssituation, späte Stentthrombosen hingegen werden oftmals nicht einmal zum betreffenden Arzt persönlich zurückgemeldet. Daraus ergibt sich ein subjektiver Handlungsdruck in Richtung einer tendenziell schlechten Einbettung mit zu geringer Stentaufweitung.

Hinzu kommt das Risiko, eines unzureichenden Feedbacks an den Behandelnden über die Folgen einer suboptimalen Stenteinbettung, da entsprechende Medikamente (wie z.B. Dual Anti Platelet Therapy Medikamente, kurz DAPT Medikamente) oftmals die negativen Folgen unterbinden, solange die Medikamente regelmäßig eingenommen werden. Derartige DAPT Medikamente sind jedoch sehr belastend für den Organismus.

Der Erfindung liegt hiervon ausgehend die Aufgabe zugrunde, ein medizinisches System zu schaffen, dass die Prozedur des Stentsetzens dahingehend unterstützt, dass sich der Stent nach einer Angioplastie trotz Stentrecoil und trotz einer ggf. vorliegenden Gefäßdurchmesseränderung aufgrund von Blutdruckschwankungen nicht mehr von der Gefässwandung lösen kann, und insbesondere das Risiko einer Dissektion (unter einer Dissektion wird eine Ruptur der Gefäßwand verstanden) vermeidbar ist, so dass nach Möglichkeit alle Patienten eine optimale Behandlungsmethode erhalten. Allen involvierten Parteien (z.B. Patient, Arzt, Hospital, Versicherung, Staat) soll insbesondere ein Werkzeug zum Erhalten, Anwenden, Sicherstellen, Einfordern und Überwachen der besten Behandlungsmethode gegeben werden.

Diese Aufgabe wird durch ein medizinisches System mit den Merkmalen des Anspruchs 1 sowie durch ein Verfahren gelöst. Vorteilhafte Ausgestaltungen des medizinischen Systems sind in den entsprechenden Unteransprüchen angegeben bzw. werden nachfolgend beschrieben.

Gemäß Anspruch 1 wird ein System zur Unterstützung der Einbettung eines vaskulären Implantats, insbesondere eines Stents, in ein Gefäß eines Patienten im Bereich einer Stenose bei einer Angioplastie offenbart, mit:
- einem Ballonkatheter, der einen in einer axialen Richtung erstreckten Ballon aufweist sowie optional ein darauf angeordneten vaskulären Implantat, insbesondere ein Stent, wobei der Ballon zum Aufweiten des Implantats, inbesondere des Stents, in radialer Richtung dehnbar ist, und wobei das System zumindest einen Sensor aufweist, der dazu ausgebildet ist, einen Istwert eines Ballonparameters zu messen und insbesondere an die Datenverarbeitungseinheit zu übermitteln,
- einer Datenverarbeitungseinheit, die dazu ausgebildet ist, folgende Daten zu erfassen: Patientendaten, Daten zur Angioplastie, Daten zur aktuellen Situation des Patienten, Stenosedaten (d.h. die Stenose charakterisierende Daten), einen Ballonparameter,
- einer Datenbankeinheit, wobei die Datenverarbeitungseinheit dazu ausgebildet ist, die Daten (insbesondere den Ballonparameter) an die Datenbankeinheit zu übermitteln, wobei die Datenbankeinheit dazu ausgebildet ist, die Daten in einer Datenbank der Datenbankeinheit zu Speichern und zu analysieren, und wobei
- die Datenbankeinheit dazu ausgebildet ist, eine multivariate Analyse der besagten Daten durchzuführen, um eine Eintrittswahrscheinlichkeit einer Komplikation bei der Angioplastie abzuschätzen und/oder eine Handlungsempfehlung für eine die Angioplastie durchführende Person zu erzeugen und über eine Benutzerschnittstelle des Systems mitzuteilen.

Das erfindungsgemäße System wird hauptsächlich bei der Implantation eines Stents mittels eines Ballonkatheters angewandt, kann jedoch auch zur späteren Postdilatation (Nachträgliche zusätzliche Expansion) mittels Ballonkatheter eines bereits implantierten Stents oder zur Implantation eines anderen vaskulären Implantats verwendet werden. Im Folgenden wird die Erfindung am Beispiel eines Stents beschrieben, ist jedoch für jedes beliebige vaskuläre Implantat geeignet.

Gemäß einer Ausführungsform des erfindungsgemäßen Systems ist vorgesehen, dass es sich bei dem Ballonparameter um einen Durchmesser des Ballons in der radialen Richtung des Ballons handelt, der quer zu der axialen Richtung des Ballons verläuft, die insbesondere mit der Katheterlängsachse zusammenfällt. Da der Ballon zum Aufweiten des Stents durch Befüllen des Ballons mit einem fluiden Medium ausgedehnt bzw. entfaltet wird, wird dieser Durchmesser auch als Aufweitdurchmesser bezeichnet.

Weiterhin ist gemäß einer bevorzugten Ausführungsform des erfindungsgemäßen Systems vorgesehen, dass die automatisch vom medizinischen System generierte Handlungsempfehlung einen Vorschlag für einen Sollwert des Ballonparameters aufweist (z.B. einen Sollwert für einen Durchmesser des Ballons in der radialen Richtung, der bei der Angioplastie zum Einbetten des Stents eingestellt werden soll).

Die besagte Person/Arzt, die bzw. der die Angioplastie durchführt, kann durch eine Einschätzung der Schwere der erwarteten Komplikationen eine optimale bzw. angepasste Behandlungsstrategie festlegen. Die Angioplastie bzw. Prozedur wird aufgezeichnet und etwaige Komplikationen werden der Datenbank der Datenbankeinheit hinzugefügt. Das medizinische System wird somit mit jedem neuen Datensatz mit Vorteil präziser.

Typische Komplikationen die auftreten können, sind z.B.
- die Dissektion aufgrund der Angioplastie
- sowie eine späte Stentthrombose aufgrund schlechten Einwachsens des Stents.

Zweckmäßigerweise wird das medizinische System bei der Herstellung mit dem neuesten überprüften und freigegebenen Vorhersagemodell zur Generierung der Handlungsempfehlung ausgestattet. Vor der Verwendung kann der Arzt bei Vorliegen einer Internetanbindung die Wahl dieses Vorhersagemodell durch eine aktuellere Version zu ersetzen.

Gemäß einer Ausführungsform des medizinischen Systems vorgesehen, dass der Ballon aus einem thermoplastischen Ballonmaterial hergestellt ist, das eine genügend hohe Druckfestigkeit bei geringer Wandstärke bietet. Bei dem Material kann es sich z.B. um ein Polymer (z.B. aus der Polyamidfamilie) oder auch um ein thermoplastisches Polyurethan handeln. Der Ballonformprozess ist vorzugsweise so ausgelegt, dass sich ein semi-compliant Ballonverhaltenergibt, welches genügend Komfort und Flexibilität in der Anwendung bietet. Unter einem semi-compliant Ballonverhalten wird im Rahmen dieser Anmeldung ein Ballonverhalten verstanden, bei dem sich der Durchmesser des Ballons noch um mindestens 10%, bevorzugt 20% gegenüber dem Nominaldurchmesser erhöht, wenn weiter Fluid zugeführt wird. Unter dem Nominaldurchmesser wird im Rahmen der Anmeldung der Designdurchmesser des Ballons verstanden. Der Nominaldurchmesser wird zusammen mit dem zugehörigen Nominaldruck für jeden Ballon festgelegt und angegeben.

Weiterhin ist gemäß einer bevorzugten Ausführungsform des medizinischen Systems vorgesehen, dass das medizinische System, insbesondere die Datenverarbeitungseinheit, dazu konfiguriert ist, nach bzw. während der Angioplastie ein Ergebnis der Angioplastie zu erfassen und an die Datenbankeinheit zu übersenden, wobei die Datenbankeinheit dazu konfiguriert ist, das Ergebnis in der Datenbank zu speichern und bei einer multivariaten Analyse für eine weitere Angioplastie zu verwenden, um insbesondere die Erfolgsaussichten der Angioplastie für eine möglichst große Patientenanzahl zu verbessern.

Bei dem besagten Ergebnis kann es sich z.B. um eine Information handeln, dass die Angioplastie erfolgreich verlaufen ist (ohne Komplikationen), oder um die Information, dass sich bei der Angioplastie eine oder mehrere Komplikationen (siehe oben) ereignet haben, wobei diese Information vorzugsweise eine Beschreibung der aufgetretenen Komplikationen enthält, oder es kann sich bei dem Ergebnis um die Information handeln, ob es in einem Zeitraum nach der Angioplastie zu einer Komplikation gekommen ist (z.B. sogenannte (sehr) späte Stentthrombose die Jahre nach der Implantation auftreten kann).

Die Güte der besagten automatisch generierten Handlungsempfehlung bzw. Vorhersage erhöht sich mit der Qualität der Daten. Daher werden Sensoren, die den Verlauf der Angioplastie präzise überwachen, besonders bevorzugt im Rahmen der vorliegenden Erfindung eingesetzt. Besonders wichtig für die Langzeitverbesserung der klinischen Ergebnisse ist die Rückmeldung akuter und späterer Probleme/Komplikationen (wie z.B. die besagte Dissektion und späte Stentthrombose).

Weiterhin ist gemäß einer bevorzugten Ausführungsform des erfindungsgemäßen medizinischen Systems vorgesehen, dass die Patientendaten eine oder mehrere der folgenden Informationen enthalten: das Alter des Patienten; eine Auflistung von Krankheiten des Patienten sowie das jeweils zugehörige Krankheitsbildes des Patienten; eine Auflistung eines oder mehrere Medikamente, die der Patient eingenommen hat (sowie insbesondere deren jeweilige Dosierung); eine Auflistung von Kontraindikationen. Bei den Kontraindikationen handelt es sich jeweils um einen Umstand des Patienten, der die Anwendung eines diagnostischen oder therapeutischen Verfahrens, insbesondere die Angioplastie, bei an sich gegebener Indikation verbietet.

Weiterhin ist gemäß einer Ausführungsform des erfindungsgemäßen medizinischen Systems vorgesehen, dass die Daten zur Angioplastie eine oder mehrere der folgenden Informationen enthalten: eine Auflistung zuvor am Patienten durchgeführter Behandlungsschritte (wie z.B. eine Vordilatation des von der Stenose betroffenen Gefäßes, eine Stentimplantation etc.); eine Auflistung von noch nicht erfolgten und nunmehr für den Patienten vorgesehenen Behandlungsschritten (z.B. Messen der Stenose, Öffnen der Stenose mittels PO-BA (für plain old balloon angioplasty, d.h., klassische Ballonangioplastie ohne medikamentenbeschichteten Ballon und ohne Stent), Implantieren eines medikamentenbeschichteten Stents (sogenannter DES für drug eluting stent), Vornahme einer Korrektur mittels Postdilatation um den Stent besser an die Arterienwandung anzupassen.

Weiterhin ist gemäß einer Ausführungsform des erfindungsgemäßen medizinischen Systems vorgesehen, dass die Daten zur aktuellen Situation des Patienten, eine oder mehrere der folgenden Informationen enthalten: einen Blutdruck des Patienten; einen Puls des Patienten; eine Auflistung aktuell eingenommener Medikamente und insbesondere deren Dosierung (z.B. Heparin, Blutdrucksenker etc.).

Gemäß einer weiteren Ausführungsform des erfindungsgemäßen medizinischen Systems ist vorgesehen, dass die Stenosedaten eine oder mehrere der folgenden Informationen enthalten: einen Ort der Stenose im Gefäß; einen Durchmesser eines gesunden Abschnitts des Gefäßes, der an die Stenose angrenzt (wird als gesunder Gefäßdurchmesser bezeichnet); eine Klassifizierung der Stenose (z.B. in vernarbte oder kalzifizierte Stenose, Symmetrie der Verengung, Re-Stenose). Von Vorteil zur Charakterisierung des Eingriffs ist auch eine Angabe zur erwarteten Abweichung vom Zieldurchmesser während des Eingriffs durch den Widerstand der Stenose gegen das Aufweiten, z.B. in Form eines Vertrauensintervalls der Messgröße Stenosendurchmesser. Dieses Vertrauensintervall ist insbesondere bei geringen Balloninnendrücken groß, da der Ballondurchmesser im Bereich der Stenose lokal vom maximal erreichten Ballondurchmesser außerhalb der Stenose stärker abweichen wird. Spätestens beim Erreichen von Balloninnendrücken, die ausreichen, die Stenose zu brechen, wird die Abweichung nach unten, die durch den Widerstand der Stenose verursacht wurde, ausgeschaltet. Ist die Ballonmembran erst einmal gespannt, bestimmt die Balloncompliance maßgeblich den Ballondurchmesser.

Bekannte Analysemethoden sind z.B. die Impedanzspektroskopie, die z.B. zwischen kalzifizierter und vernarbter Stenose unterscheiden kann. Weiterhin kann zur Unterscheidung zwischen kalzifizierter und vernarbter Stenose eine Stenosencompliance gemessen werden. Unter Stenosencompliance wird das Maß verstanden, wie sich der Stenosendurchmesser durch eine Druckerhöhung von innen ändert.

Hierzu kann das betroffene Gefäß distal von der Stenose durch einen Ballon für die Messung der Stenosencompliance Messung verschlossen werden. Als Druckänderung nutzt man dann den Pulsschlag im Gefäß und beobachtet die Durchmesseränderung des Gefäßes oder man misst den Volumenstrom. Zusätzlich kann ein zweiter Ballon das Gefäß auch proximal abriegeln und man beaufschlagt den abgeriegelten Bereich mit einem hydraulischen Druck und beobachtet die Formänderung. Alternativ verschließt man das Gefäß distal und proximal der Stenose und pumpt ein Volumen in den abgeriegelten Bereich und misst dann den Druckanstieg. Diese Messungen sollten unterschiedliche Verläufe für vernarbte und kalzifizierte Stenosen ergeben, so dass eine Unterscheidung dieser Stenosetypen möglich ist.

Die Stenosencompliance kann z.B. auch durch das Messen des Volumenflusses in einen Ballon in Abhängigkeit vom Druck charakterisiert werden. Das funktioniert nur so lange wie der Ballondurchmesser grösser ist als der Stenosendurchmesser. Ist der Ballon nach erfolgtem Druckanstiegs auch kleiner als der Gefäßdurchmesser, wird nur die Durchmesseränderung im Bereich der Stenose erfasst, die während der Druckbeaufschlagung im Kontakt mit dem Ballon war.

Weiterhin ist gemäß einer Ausführungsform des medizinischen Systems vorgesehen, dass die Datenverarbeitungseinheit dazu ausgebildet ist, einen für die Angioplastie ausgewählten Sollwert des Ballonparameters, der bei der Angioplastie verwendet werden soll bzw. einzustellen ist, zu erfassen (z.B. durch eine Eingabe eines Benutzers des Systems) und an die Datenbankeinheit zu übermitteln. Zur Überwachung der Angioplastie ist der Durchmesser des Ballons der wichtigste Parameter beim Setzen bzw. Einbetten des Stents oder beim Nachdilatieren des Stents.

Gemäß einer weiteren Ausführungsform des erfindungsgemäßen medizinischen Systems ist vorgesehen, dass die Datenbankeinheit dazu konfiguriert ist, vor der Einbettung des Stents im Rahmen der Angioplastie zu prüfen, ob die besagte Eintrittswahrscheinlichkeit einer Komplikation für den ausgewählten Sollwert des Ballonparameters (z.B. Sollwert des Aufweitdurchmessers) unterhalb eines vordefinierten Schwellenwertes liegt, so dass der prozedurale Erfolg absehbar ist.

Weiterhin ist gemäß einer Ausführungsform des erfindungsgemäßen medizinischen Systems vorgesehen, dass das System eine Inflationspumpe zum Befüllen des Ballons mit einem fluiden Medium aufweist, wobei das System (insbesondere die Datenverarbeitungseinheit) dazu ausgebildet ist, einen mittels der Inflationspumpe erzeugten Druck des Mediums im Ballon automatisch so zu regeln, dass ein Istwert des Ballonparameters (insbesondere der Durchmesser des Ballons in der radialen Richtung des Ballons) den ausgewählten Sollwert des Ballonparameters bzw. Durchmessers erreicht.

Mit anderen Worten regelt (oder steuert) hier das System bzw. die Datenbankeinheit die Inflationspumpe. Die Angioplastie nach Positionieren des Ballons am vorgesehenen Ort wird automatisch durchführt. Vorzugsweise wird hierbei der Ballonparameter/Aufweitdurchmesser direkt oder indirekt laufend überwacht.

Insbesondere für den Fall, dass der Ballonparameter bzw. Aufweitdurchmesser des Ballons nicht mit einem dezidierten Sensor erfassbar ist, kann zum Beispiel auch der im Ballon herrschender Druck eines in den Ballon eingefüllten fluiden Mediums als Ballonparameter verwendet werden, wobei das System hier bevorzugt dazu ausgebildet ist, den Druck sowie insbesondere einen Volumenstrom in den Ballon zu messen und über eine Benutzerschnittstelle des Systems der die Angioplastie durchführenden Person/Arzt zur Überwachung einer Aufweitung des Stents anzuzeigen. Die Abweichung des tatsächlichen Volumenstroms im Vergleich zum in-vitro Volumenstrom desselben Katheters für ein vorgegebenes Druckintervall ist ein Indikator für die Präzision des erreichten Durchmessers. Bei noch nicht aufgebrochener Stenose ist das tatsächliche Kontrastmediumvolumen im Ballon reduziert, da die Stenose den Ballon am Auffalten hindert. Der Stenosegrad kann damit schon bei geringem Druck bestimmt werden. Ebenso korreliert die Stenosencompliance bzw. der Fortschritt der Angioplastie mit dem Verhältnis von Zunahme des Ballonvolumens zu Druckanstieg im Ballon.

Weiterhin ist gemäß einer bevorzugten Ausführungsform des erfindungsgemäßen Systems vorgesehen, dass das System insbesondere die Datenverarbeitungseinheit dazu konfiguriert ist, eine Dehnung des Ballons über einen gesunden Gefäßdurchmesser des Patienten hinaus zu ermitteln und über eine Benutzerschnittstelle des Systems der die Angioplastie durchführenden Person anzuzeigen, wobei der gesunde Gefäßdurchmesser ein Durchmesser eines sich an die Stenose anschließenden gesunden Abschnitts des Gefäßes ist (siehe auch oben).

Weiterhin ist gemäß einer bevorzugten Ausführungsform des erfindungsgemäßen Systems vorgesehen, dass das System, insbesondere die Datenverarbeitungseinheit, dazu ausgebildet ist, zum Ermitteln der Dehnung eine Differenz zwischen einem Istwert des Ballonparameters bzw. des Durchmesser des Ballons in der radialen Richtung und dem gesunden Gefäßdurchmesser oder eine zu dieser Differenz proportionale Größe (z.B. die auf den gesunden Gefäßdurchmesser normierte Differenz) zu berechnen und über eine Benutzerschnittstelle des Systems darzustellen.

Gemäß einer weiteren Ausführungsform des erfindungsgemäßen medizinischen Systems ist vorgesehen, dass das System dazu ausgebildet ist, die Dehnung des Ballons ab dem Moment in dem der Istwert des Ballonparameters (hier Aufweitdurchmesser, d.h., Durchmesser des Ballons in der radialen Richtung des Ballons) und der gesunde Gefäßdurchmesser zusammenfallen (d.h., der Ballon und der gesunde Abschnitt des Gefäßes setzen sich stetig fort) auf Null zu setzen und die dann fortschreitende Dehnung des Ballons über den gesunden Gefäßdurchmesser hinaus als Prozentwert des gesunden Gefäßdurchmessers (oder in sonstiger Weise) über eine Benutzerschnittstelle des Systems anzuzeigen. Der Prozentwert ist hierbei also die besagte Differenz geteilt durch den gesunden Gefäßdurchmesser, siehe oben.

Weiterhin ist gemäß einer bevorzugten Ausführungsform des erfindungsgemäßen medizinischen Systems vorgesehen, dass das System dazu ausgebildet ist, ein visuelles Signal und/oder ein akustisches Signal über eine Benutzerschnittstelle des Systems auszugeben, wenn der Istwert des Ballonparameters (z.B. der besagte Aufweitdurchmesser) den Sollwert des Ballonparameters erreicht. Weiterhin kann gemäß einer Ausführungsform (insbesondere zusätzlich) vorgesehen sein, dass das System dazu ausgebildet ist ein visuelles Warnsignal und/oder ein akustisches Warnsignal über eine Benutzerschnittstelle des Systems auszugeben, wenn der Istwert des Ballonparameters (z.B. der Aufweitdurchmesser) den Sollwert des Ballonparameters überschreitet, so dass insbesondere die die Angioplastie durchführende Person vor der Zunahme z.B. des Rupturrisikos deutlich gewarnt wird.

Das zum Aufweiten des Stents benutzte erfindungsgemäße medizinische System hilft somit dem Anwender bzw. der die Angioplastie vornehmenden Person beim Ansteuern der empfohlenen Überdehnung des Stents (um 10-20%) im Vergleich zum gesunden Gefäßdurchmesser.

Gemäß einer Ausführungsform des medizinischen Systems ist vorgesehen, dass dieses eine bildgebende Einheit (z.B. ein Röntgengerät) zum Überwachen der Einbettung des Stents aufweist.

Gemäß einer bevorzugten Ausführungsform des erfindungsgemäßen medizinischen Systems ist es weiterhin vorgesehen, dass das System, insbesondere die Datenbankeinheit, dazu ausgebildet ist, für den Sollwert des Ballonparameters die besagte Eintrittswahrscheinlichkeit einer Komplikation für den Patienten in Abhängigkeit einer oder mehrerer der folgenden Parameter abzuschätzen: gesunder Gefäßdurchmesser, Stenosendurchmesser, Lage (Symmetrie) des Stenosendurchmesser zum Gefässdurchmesser , Morphologie der Stenose und Stenosenlänge.

Weiterhin ist gemäß einer bevorzugten Ausführungsform des erfindungsgemäßen medizinischen Systems vorgesehen, dass das System, insbesondere die Datenbankeinheit, dazu ausgebildet ist (z.B. anhand der Ergebnisse von Angioplastien, der Klassifizierung der Stenosen und der gewählten Sollwerte des Ballonparameters bzw. Durchmessers) eine Korrelation zwischen dem Sollwert des Ballonparameters und dem Ergebnis der Angioplastie zu ermitteln, wobei die Datenbankeinheit dazu ausgebildet ist, die Korrelation zur Anpassung der besagten Handlungsempfehlung zu verwenden. Insofern werden also bei der jeweiligen Angioplastie gewonnene bzw. erfasste Daten zur Datenbankeinheit zurückgemeldet, um insbesondere zu prüfen, ob das multivariate Modell die Zukunft genügend genau vorhersagt.

Das erfindungsgemäße medizinische System setzt nicht notwendigerweise voraus, dass der besagte Ballonparameter/ Durchmesser direkt mittels eines dezidierten Sensors gemessen wird. So ist gemäß einer Ausführungsform der vorliegenden Erfindung auch denkbar, dass das medizinische System dazu ausgebildet ist, einen Druck des im Ballon befindlichen fluiden Mediums z.B. am proximalen Kathetereingang des Ballonkatheters zu messen und hierauf basierend einen Erwartungsbereich für den Ballonparameter bzw. Durchmesser des Ballons anzugeben. Idealerweise ist der Ballon so stark ausgelegt, dass sein Nominaldurchmesser erst nach Brechen der Stenose überschritten wird. Dieser Öffhungsdruck liegt für koronare Arterien bei ca. 14 bar. Vor dem Brechen der Stenose wird der tatsächliche Durchmesser des Ballons im Bereich der Stenose kleiner sein, als der auf der Compliance-Data Card angegebene, welche den Zusammenhang zwischen Ballondurchmesser und Balloninnendruck in vitro wiederspiegelt.

In einer alternativen Version des medizinischen Systems wird das in-vitro gemessene und damit erwartete Ballonvolumen mit dem realen in-vivo gemessenen Volumenstrom für jeden Balloninnendruck verglichen. Die radiale und axiale Ausdehnung eines Ballons in Abhängigkeit vom hydraulischen Druck und dem Volumenstrom ist in vitro hochgradig reproduzierbar. Eine Änderung des Druckes bei gleichem Volumenstrom impliziert einen Gegendruck, der Ballon wird durch die Stenose an der Entfaltung gehindert. Sind diese Volumina nahezu identisch wird auch der Durchmesser des Ballons (Ballonparameter) in-vivo auf der gesamten zylindrischen Länge des Ballons mit dem Durchmesser des Ballons in-vitro übereinstimmen.

Die genaueste Durchmessermessung erfolgt vorzugsweise durch zumindest einen Sensor im Bereich des Ballons. Diese Messung kann auf verschiedene Weise mit physikalischen Prinzipien ermittelt werden.

Gemäß einer Ausführungsform des erfindungsgemäßen medizinischen Systems ist vorgesehen, dass der Sensor ein auf dem Ballon angeordneter Dehnungssensor ist, wobei der Dehnungssensor vorzugsweise auf einem zylindrischen Abschnitt des Ballons angeordnet ist. Beide Bauteile, d.h., der Ballon und der Dehnungssensor, sind bevorzugt für eine Dehnung von bis zu mindestens 15% idealerweise >30% und einer Auflösung von +/-5% (vorzugsweise +/-1%) ausgelegt, ohne zu delaminieren oder einen Leitungsbruch zu riskieren.

Gemäß einer Ausführungsform des erfindungsgemäßen medizinischen Systems ist vorgesehen, dass der Ballonkatheter mit dem Sensor verbundene elektrische Leiter aufweist, über die der Dehnungssensor mit elektrischer Energie versorgbar ist.

Weiterhin ist gemäß einer Ausführungsform des medizinischen Systems vorgesehen, dass das System zur Bereitstellung der elektrischen Energie eine Energiequelle aufweist, die außerhalb des Körpers des Patienten anordenbar ist. Bei der Energiequelle kann es sich aber auch um einen Energiespeicher (z.B. Batterie) handeln, der auf dem Ballonkatheter angeordnet ist bzw. dazu ausgebildet ist, im Körper des Patienten angeordnet zu werden.

Weiterhin besteht die Möglichkeit, dass der Ballonkatheter eine mit dem Sensor verbundene Induktionsspule aufweist, so dass elektrische Energie kabellos über die Induktionsspule zum Sensor übertragbar ist.

Weiterhin ist der Sensor gemäß einer Ausführungsform der Erfindung dazu ausgebildet, ein Ausgangssignal bereitzustellen, das dem Istwert des Ballonparameters bzw. Durchmesser des Ballons in der radialen Richtung des Ballons entspricht bzw. in diesen Ballonparameter umrechenbar ist, wobei vorzugsweise die Datenverarbeitungseinheit zum Umrechnen des Ausgangssignals in den Istwert des Ballonparameters (insbesondere Durchmesser des Ballons in radialer Richtung) konfiguriert ist.

Weiterhin ist gemäß einer Ausführungsform des Systems vorgesehen, dass das System dazu konfiguriert ist, das Ausgangssignal des Sensors über eine Datenleitung oder über eine kabellose Verbindung (z.B. Funk oder Ultraschall) zur Datenverarbeitungseinheit zu übermitteln.

Weiterhin ist gemäß einer Ausführungsform des Systems vorgesehen, dass die Datenverarbeitungseinheit einen Prozessor aufweist, auf dem ein Algorithmus ausgeführt wird, der das Ausgangssignal des Sensors zum Ermitteln des Istwerts des Ballonparameters bzw. des Durchmessers des Ballons in der radialen Richtung des Ballons ausführt, wobei der Prozessor auf dem Ballonkatheter angeordnet sein kann, so dass er insbesondere innerhalb des Körpers des Patienten anordenbar ist, oder wobei der Prozessor dazu eingerichtet und vorgesehen sein kann, außerhalb des Körpers des Patienten angeordnet zu werden.

Gemäß einer Ausführungsform der Erfindung ist vorgesehen, dass der Sensor ein Dehnungsmesstreifen ist, wobei der Dehnungsmessstreifen so ausgebildet ist, dass eine Änderung eines elektrischen Widerstandes eines Leiters (insbesondere Leiterbahn) des Dehnungsmessstreifens ein Maß für die Dehnung des Leiters ist, wobei vorzugsweise der Dehnungsmessstreifen so auf dem Ballon angeordnet ist, dass die Dehnung proportional zur Umfangsänderung bzw. Durchmesseränderung des Ballons ist. Auf diese Weise kann der Sensor ein Ausgangssignal bereitstellen, dass kennzeichnend für den Istwert des Durchmessers des Ballons in der radialen Richtung des Ballons ist.

Alternativ hierzu kann der Sensor dazu ausgebildet sein, als Maß für den Ballonparameter bzw. für den Durchmesser des Ballons in der radialen Richtung des Ballons eine Änderung des elektrischen Widerstandes einer Flüssigkeitssäule im Ballon zu erfassen, wobei das in den Ballon eingefüllte fluide Medium elektrisch leitfähig ist.

Weiterhin kann alternativ hierzu der Sensor dazu ausgebildet sein, als Maß für den Ballonparameter bzw. für den Durchmesser des Ballons in der radialen Richtung des Ballons eine durch Dehnung des Ballons erzeugte Änderung einer Fläche und einer Stärke einer Ballonmembran des Ballons kapazitiv zu messen.

Ein weiterer Aspekt der Erfindung betrifft ein Verfahren zur datengestützten Optimierung der automatischen Abschätzung von Eintrittswahrscheinlichkeiten von Komplikationen und/oder der automatischen Erzeugung von Handlungsempfehlungen im Rahmen von Angioplastien unter Verwendung eines erfindungsgemäßen medizinischen Systems, wobei mittels der Datenverarbeitungseinheit zumindest die folgendem Daten erfasst werden: Patientendaten, Daten zur Angioplastie, Daten zur aktuellen Situation des Patienten, Stenosedaten, ein Ballonparameter, wobei die Daten an die Datenbankeinheit übermittelt werden, und wobei die Datenbankeinheit dazu ausgebildet ist, die Daten in einer Datenbank zu Speichern und zu analysieren, und wobei mittels der Datenbankeinheit eine multivariate Analyse der besagten Daten durchgeführt wird, um eine Eintrittswahrscheinlichkeit einer Komplikation bei einer durchzuführenden Angioplastie abzuschätzen und/oder wobei eine Handlungsempfehlung für eine die Angioplastie durchführende Person automatisch erzeugt wird und der Person über eine Benutzerschnittstelle des medizinischen Systems angezeigt wird.

Gemäß einer Ausführungsform des Verfahrens handelt es sich bei dem Ballonparameter um einen Durchmesser des Ballons in der radialen Richtung des Ballons.

Gemäß einer weiteren Ausführungsform des Verfahrens weist die Handlungsempfehlung einen Vorschlag für einen Sollwert des Ballonparameters auf, der bei der Angioplastie einzustellen ist (z.B. Durchmesser des Ballons in der radialen Richtung, auf den der Ballon bei der Angioplastie zu dehnen ist).

Gemäß einer Ausführungsform des Verfahrens wird beim Aufweiten des auf dem Ballon angeordneten Stents durch Dehnen des Ballons in radialer Richtung des Ballons ein Istwert des Ballonparameters (insbesondere der besagte Durchmesser des Ballons) mittels des Sensors gemessen, wobei vorzugsweise das Dehnen des Ballons beendet wird und/oder ein Signal angezeigt wird, wenn der Istwert des Ballonparameters den Sollwert des Ballonparameters erreicht. Vorzugsweise übersteigt der Sollwert des Durchmessers den gesunden Gefäßdurchmesser um 5% bis 20%, bevorzugt 10% bis 20% oder insbesondere 8%.

Zum Bestimmen des gesunden Gefäßdurchmessers kann ein bildgebendes Verfahren verwendet werden. Der gesunde Gefäßdurchmesser kann auch bestimmt werden, indem der Ballon gedehnt wird, bis er einen umlaufenden Kontakt zur Gefäßwand eines gesunden Abschnitts des Gefäßes aufweist, der an die Stenose angrenzt, wobei der zum Zeitpunkt des Kontakts vorliegende Durchmesser des Ballons als gesunder Gefäßdurchmesser bzw. Referenzdurchmesser verwendet wird.

Gemäß einer Ausführungsform des Verfahrens ist vorgesehen, dass der Ballon kontinuierlich oder schrittweise von der besagten Person oder automatisch gedehnt wird (z.B. mittels einer Inflationspumpe), wobei der Istwert des Ballonparameters (insbesondere besagter Durchmesser des Ballons) laufend bestimmt wird und angezeigt wird, wobei insbesondere ein Warnsignal angezeigt wird, wenn der Istwert des Ballonparameters den Sollwert des Parameters übersteigt, oder wobei das Dehnen des Ballons geregelt wird, so dass der Istwert des Ballonparameters (insbesondere besagter Durchmesser) den Sollwert des Ballonparameters automatisch anstrebt.

Weiterhin wird gemäß einer Ausführungsform des Verfahrens vorgesehen, dass ein für die Angioplastie ausgewählter Sollwert des Ballonparameters, der bei der Angioplastie einzustellen ist, mittels der Datenverarbeitungseinheit erfasst und an die Datenbankeinheit übermittelt, so dass insbesondere der Sollwert zusammen mit den weiteren Daten zur Verbesserung der multivariaten Analyse herangezogen werden kann.

Weiterhin ist gemäß einer Ausführungsform des Verfahrens vorgesehen, dass mittels der Datenbankeinheit vor der Angioplastie geprüft wird, ob die besagte Eintrittswahrscheinlichkeit einer Komplikation für den Sollwert des Ballonparameters unterhalb eines vordefinierten Schwellenwertes liegt.

Weiterhin ist gemäß einer Ausführungsform des Verfahrens vorgesehen, dass mittels einer Inflationspumpe ein fluides Medium in den Ballon eingefüllt wird, wobei der solchermaßen im Ballon erzeugte Druck des fluiden Mediums mit Hilfe der Inflationspumpe automatisch so geregelt wird, dass ein wiederholt gemessener Istwert des Ballonparameters (z.B. besagter Durchmesser des Ballons in der radialen Richtung des Ballons) den Sollwert des Ballonparameters erreicht.

Weiterhin ist gemäß einer Ausführungsform des Verfahrens vorgesehen, dass eine Dehnung des Ballons über einen gesunden Gefäßdurchmesser des Patienten hinaus ermittelt wird und über eine Benutzerschnittstelle des Systems angezeigt wird, wobei der gesunde Gefäßdurchmesser ein Durchmesser eines sich an die Stenose anschließenden gesunden Abschnitts des Gefäßes ist.

Weiterhin ist gemäß einer Ausführungsform des Verfahrens vorgesehen, dass die Dehnung des Ballons ab dem Moment, in dem der Istwert des Ballonparameters bzw. des besagten Durchmessers des Ballons und der gesunde Gefäßdurchmesser zusammenfallen, automatisch auf Null gesetzt wird und die dann fortschreitende Dehnung des Ballons über den gesunden Gefäßdurchmesser hinaus als Prozentwert des gesunden Gefäßdurchmessers über die Benutzerschnittstelle angezeigt wird.

Weiterhin ist gemäß einer Ausführungsform des Verfahrens vorgesehen, dass ein visuelles Signal und/oder ein akustisches Signal über eine Benutzerschnittstelle ausgegeben wird, wenn der Istwert des Ballonparameters (insbesondere der besagte Durchmesser des Ballons) den Sollwert des Ballonparameters erreicht; und/oder wobei ein visuelles Warnsignal und/oder ein akustisches Warnsignal über eine Benutzerschnittstelle ausgegeben wird, wenn der Istwert des Ballonparameters den Sollwert des Ballonparameters überschreitet.

Weiterhin ist gemäß einer Ausführungsform des Verfahrens vorgesehen, dass mittels der Datenbankeinheit automatisch für den Sollwert des Ballonparameters die besagte Eintrittswahrscheinlichkeit einer Komplikation für den Patienten in Abhängigkeit einer oder mehrerer der folgenden Parameter abgeschätzt wird: des gesunden Gefäßdurchmessers, des Stenosendurchmessers, der Stenosenlänge.

Weiterhin ist gemäß einer Ausführungsform des Verfahrens vorgesehen, dass mittels der Datenbankeinheit eine Korrelation zwischen dem Sollwert des Ballonparameters und dem Ergebnis der Angioplastie zu ermittelt wird (siehe auch oben), wobei die Datenbankeinheit die Korrelation zur Anpassung der besagten Handlungsempfehlung verwendet.

Nachfolgend sollen Merkmale, Vorteile und Ausführungsformen der vorliegenden Erfindung anhand der Figuren erläutert werden. Es zeigt:
- Fig. 1: eine schematische Darstellung einer Ausführungsform eines erfindungsgemäßen medizinischen Systems; und
- Fig. 2: eine schematische grafische Darstellung des Aufweitdurchmessers des Ballons bei der Angioplastie.

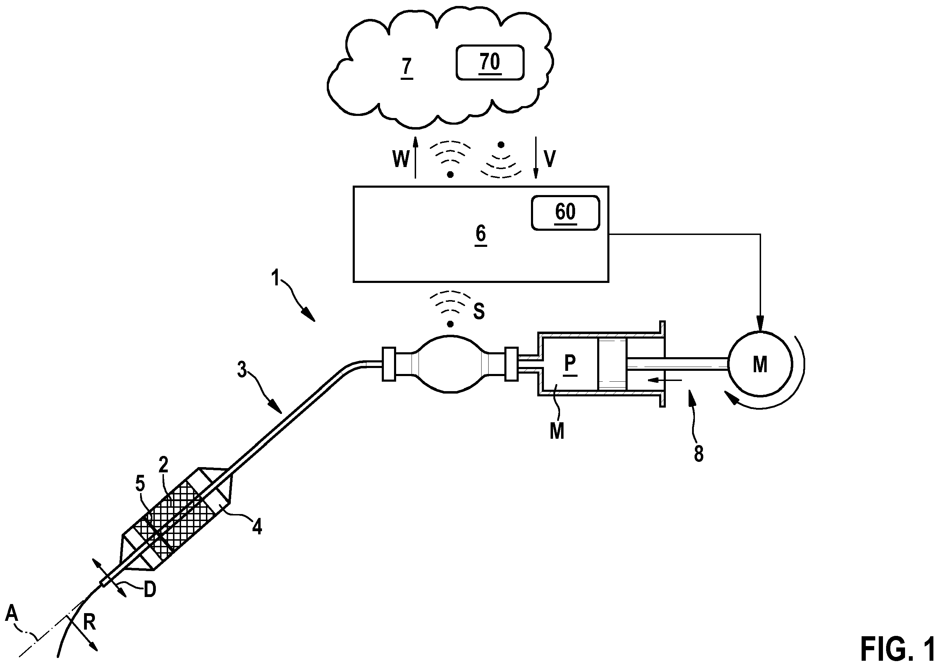

Figur 1 zeigt eine Ausführungsform eines erfindungsgemäßen medizinisches Systems 1 zur Unterstützung der Einbettung eines Stents 2 in ein Gefäß eines Patienten im Bereich einer Stenose während einer Angioplastie, wobei das System 1 zumindest aufweist: einem Ballonkatheter 3 zum Einbetten des Stents 2 in das Gefäß, derart, dass die Stenose aufgeweitet wird, wobei der Ballonkatheter 3 einen in einer axialen Richtung erstreckten Ballon 4 und den darauf festgelegten Stent 2 aufweist, wobei der Ballon 4 zum Aufweiten des Stents in radialer Richtung R dehnbar ist, und wobei das System 1 zumindest einen Sensor 5 aufweist, der dazu ausgebildet ist, einen Istwert eines Ballonparameters zu messen und insbesondere an die Datenverarbeitungseinheit zu übermitteln, wobei es sich hier bei dem Ballonparameter exemplarisch um einen Durchmesser D des Ballons 4 in der radialen Richtung R des Ballons 4 handelt. Da der Ballon 4 zum Aufweiten des Stents 2 durch Befüllen des Ballons mit einem fluiden Medium M ausgedehnt bzw. entfaltet wird, wird dieser Durchmesser D hierin auch als Aufweitdurchmesser D bezeichnet.

Weiterhin weist das medizinische System eine Datenverarbeitungseinheit 6 auf, die dazu ausgebildet ist, folgende Daten zu erfassen: Patientendaten, Daten zur Angioplastie, Daten zur aktuellen Situation des Patienten, die Stenose charakterisierende Daten, einen Ballonparameter. Die Datenverarbeitungseinheit 6 kann z.B. durch einen Rechner mit einem Mikroprozessor gebildet sein, auf dem ein die Funktionen der Datenverarbeitungseinheit 6 darstellendes Programm ausgeführt wird. Bei dem Rechner kann es sich um alle erdenklichen Computer handeln, z.B. einen Desktop-Computer, einen Laptop, ein Tablet-PC, ein Smartphone. Weiterhin weist das medizinische System 1 eine Datenbankeinheit 7 auf, wobei die Datenverarbeitungseinheit 6 dazu ausgebildet ist, die Daten an die Datenbankeinheit 7 zu übermitteln, wobei die Datenbankeinheit 7 dazu ausgebildet ist, die Daten in einer Datenbank 70 zu Speichern und/oder zu analysieren. Die Datenbankeinheit 7 kann z.B. durch einen oder mehrere vernetzte Computer und eine darauf ausgeführtes Programm gebildet sein.

Schließlich ist die Datenbankeinheit 7 weiterhin dazu ausgebildet, eine multivariate Analyse der besagten Daten durchzuführen um eine Eintrittswahrscheinlichkeit einer Komplikation bei der Angioplastie abzuschätzen und/oder eine Handlungsempfehlung für eine die Angioplastie durchführende Person zu erzeugen und der Person über eine Benutzerschnittstelle 60 des Systems 1 anzuzeigen.

Grundsätzlich kann z.B. die Datenverarbeitungseinheit 6 über eine Internetverbindung bzw. ein Internetprotokoll mit der Datenbankeinheit 7 kommunizieren.

Besonders bevorzugt entspricht die besagte Handlungsempfehlung einem Vorschlag für einen Sollwert des Ballonparameters bzw. des Durchmessers D des Ballons 4 in der radialen Richtung R, der bei der Angioplastie einzustellen ist. Hierbei kann die Einstellung manuell durch die Person vorgenommen werden oder nach Annahme des Vorschlags (z.B. durch eine entsprechende Benutzereingabe) automatisch durch das System 1 durchgeführt werden, z.B. mittels einer Inflationspumpe 8, siehe unten.

Die Erfindung stellt somit mit andere Worten insbesondere eine Schnittstelle im Katheterlabor zur Verfügung, die alle relevanten Informationen (insbesondere hinsichtlich Mensch,. Maschine, Methode, Werkzeug, Millieu, Material) der Angioplastieprozedur erfasst und Ergebnisse bzw. Komplikationen an die eine vorzugsweise standortübergreifende Datenbank 70 der Datenbankeinheit sendet.

Ein mittels der Datenbankeinheit 7 ausgeführtes Programm wertet vorzugsweise die gesammelten Daten adressatengerecht aus.

Hinsichtlich des Patienten, an dem die Angioplastie vorgenommen werden soll bzw. vorgenommen wird, kann z.B. mittels der Datenbankeinheit 7 vor der Angioplastie eine prozedurale Erfolgswahrscheinlichkeit für die geplante Angioplastie ermittelt werden, z.B. auf der Basis einer Erfolgsbilanz der die Angioplastie durchführenden Person oder Hospitals. Nach der Durchführung der Angioplastie kann mittels der Datenbankeinheit 7 z.B. eine Vorhersage des klinischen Erfolgs der durchgeführten Angioplastie aufgrund eines an die Datenbankeinheit 7 übermittelten Fallberichts sowie durch Rückmeldung des Wohlbefindens des Patienten vorgenommen werden

Die die Angioplastie durchführende Person bzw. der Arzt kann das erfindungsgemäße System 1 mit Vorteil zur preoperative Planung verwenden. Hierbei kann z.B. eine prozedurale Erfolgswahrscheinlichkeit für einen Vorschlag, wie die Angioplastie durchzuführen ist (sogenannte best practice), anhand der in der Datenbank 70 gesammelten Fallberichte zu vergangenen Angioplastien vorgenommen werden.

Weiterhin ist die Datenverarbeitungseinheit 6 vorzugsweise dazu konfiguriert, der die Angioplastie durchführenden Person bzw. einer Inflationspumpe 8 während der Angioplastie Anweisungen zu geben, wie der Ballon 4 zu dilatieren ist. Die Anweisung bzw. Handlungsanweisung kann z.B. das Einstellen des Zielballoninnendrucks betreffen. Im Gegenzug kann die Datenverarbeitungseinheit 6 dazu konfiguriert sein, einen Vertrauensbereich für den erreichten Durchmesser des Ballons in-vivo anzugeben und z. B. auch die aktuellen Risiken für das Eintreten einer späten Stentthrombose und/oder einer Dissektion (Gefäßruptur).

Weiterhin kann die Datenbankeinheit 7 dazu konfiguriert sein, hinsichtlich der postoperativen Planung Vorschläge für Nachsorgeunteruntersuchungen vorzuschlagen, z.B. basierend auf einem Vergleich des dem Patienten zugeordneten Fallberichts mit bereits in der Datenbank 70 vorhandenen Fallberichten bzw. historischen Daten. Weiterhin kann die Datenbankeinheit 70 dazu konfiguriert sein, dem Arzt die persönliche aktuelle Erfolgsbilanz für durchgeführte Angioplastien mitzuteilen.

Schließlich kann der Arzt bzw. die die Angioplastie durchführenden Person die Datenbankeinheit 7 dazu verwenden, gespeicherte Daten zu einem beliebigen Zeitpunkt bereitzustellen wobei die Datenbank 70 mit Vorteil eine Nachverfolgbarkeit der gespeicherten bzw. gewonnenen Daten für wissenschaftliche Veröffentlichungen sicherstellen kann.

Für den Hersteller des bei der Angioplastie verwendeten Ballonkatheters 3 eröffnet das erfindungsgemäße medizinische System mit Vorteil die Möglichkeit eine vereinfachte Ursachensuche für Beschwerden hinsichtlich der Funktionen des Ballonkatheters 3 durchzuführen, z.B. in dem die Datenbankeinheit 7 dazu konfiguriert ist, die Inflationszeit und die Deflationszeit des Ballons 4 bei Inflationsproblemen zu erfassen. Weiterhin kann z.B. ein maximaler Druck bei einem vorzeitigen Platzen des Ballons in der Datenbank 70 gespeichert werden. Letztlich ermöglicht das Speichern der katheterbezogenen Daten mit Vorteil einen Performance-Vergleich der einzelnen Kathetereigenschaften (z.B. von verschiedenen Herstellern bzw. zwischen verschiedenen Modellen).

Für das jeweilige Hospital, in dem die Angioplastie stattfindet, eröffnet das erfindungsgemäße System 1 grundsätzlich die Möglichkeit die Datenbankeinheit 7 so zu konfigurieren, dass die Erfolgsbilanz der betreffenden Abteilung im Vergleich zu anderen Hospitälern aus den Daten extrahierbar ist. Dies erlaubt insbesondere die für eine Angioplastie benötigte Zeitdauer im Katheterlabor zu optimieren und gestattet die Notwendigkeit der Angioplastie einfach nachzuweisen und zu dokumentieren, wenn beispielsweise die Kathetersensoren durch eine Messung der fraktionellen Flussreserve (FFR Messung) den Beleg für die Notwendigkeit der Angioplastie bzw. des Setzen eines Stents schaffen.

Schließlich eröffnet das erfindungsgemäße System 1 der jeweiligen für den Patienten zuständigen Krankenkasse die Möglichkeit hinsichtlich der Angioplastie eine beste Behandlungsmethode ("Best-Practice") vorzuschreiben, die aufgrund der Auswertung der Fallberichte zu den einzelnen Angioplastien sowie den entsprechenden Nachsorgeuntersuchungen gewonnen werden kann. Schließlich kann anhand der in der Datenbank 70 gesammelten Daten auch eine Überwachung des Einhaltens der festgelegten Best-Practice-Prozedur vorgenommen werden.

Das erfindungsgemäße System 1 ist also vorzugsweise dazu ausgebildet, den einzelnen an der Angioplastie beteiligten Parteien, also der die Angioplastie durchführenden Person (Arzt), dem Hospital, dem Hersteller und der Krankasse gezielte Informationen und Auswertungen der bei der jeweiligen Angioplastie gesammelten Daten zur Verfügung zu stellen. Das erfindungsgemäße System 1 ermöglicht auf diese Weise insbesondere einen Datenaustausch zwischen den besagten Personen/Institutionen sowie für alle Beteiligten eine Optimierung der Angioplastieprozedur, und zwar mit Vorteil angepasst an den individuellen Patienten.

Gemäß einer Ausführungsform des erfindungsgemäßen medizinischen Systems 1 bzw. des hierdurch ermöglichten Verfahrens ist, wie eingangs bereits dargelegt, vorgesehen, dass vor der Dilatation des Ballons 4 auf Basis der historischen (mittels der Datenbankeinheit gesammelten) Daten mittels einer multivariaten Analyse oder mittels eines neuronalen Netzwerks die Wahrscheinlichkeit des Eintretens von Komplikationen in Abhängigkeit von z.B. dem Aufweitdurchmesser D erstellt werden.

Der Arzt kann in der Folge durch seine Einschätzung der Schwere der erwarteten Komplikationen die optimale Behandlungsstrategie festlegen. Die Prozedur wird aufgezeichnet und Komplikationen werden der Datenbank 70 hinzugefügt. Das medizinische System 1 wird somit mit jedem neuen Datensatz präziser.

Die relevanten Daten können z.B. direkt vor Ort, d.h., am Ort der Angioplastie durch die Datenverarbeitungseinheit 6 gesammelt werden (z.B. durch Benutzereingabe in eine Benutzschnittstelle 60 des Systems 1 bzw. der Datenverarbeitungseinheit).

Die Daten betreffen dabei z.B. den Patienten, an dem die Angioplastie durchgeführt werden soll, insbesondere seiner Vorgeschichte, z. B: Alter des Patienten, Krankheiten und zugehöriges Krankheitsbild, eingenommene Medikamente sowie insbesondere deren Dosierung, vorliegende Kontraindikationen.

Hinsichtlich der Angioplastie, können die gesammelten Daten z.B. Informationen hinsichlich vorheriger Behandlungsschritte (z.B. Vordilatation, Stentimplantation, etc.) sowie einer vorgesehene Strategie zur Behandlung (Messen, Öffnen (Ballonangioplastie POBA), Stabilisieren mit Stents, insbesondere wirkstofffreisetzenden Stents (Drug Eluting Stents oder DES), Korrigieren der Stentaufweitung mittels Postdilatation (POBA)) enthalten.

Weiterhin können die von der Datenbankeinrichtung erfassten bzw. zu erfassenden Daten zur aktuellen Situation des Patienten folgende Informationen umfassen: Blutdruck des Patienten, Puls des Patienten, Medikamentenversorgung (Heparin, Blutdrucksenker, etc.)

Hinsichtlich der zu behandelnden Stenose erfasst die Datenbankeinheit bevorzugt Daten wie z.B.: genauer Ort der Stenose, gesunder Gefäßdurchmesser, Länge und minimaler Durchmesser der Stenose, Klassifizierung der Stenose in vernarbte Stenose, kalzifizierte Stenose, oder Restenose), die Art der Stenosenqualifizierung und falls bekannt Vertrauensintervall der Messgröße.

Bekannte Analysemethoden sind z.B. die Impedanzspektroskopie, die z.B. hinsichtlich der Stenose zwischen kalzifiziert und vernarbt unterscheiden kann.

Zur Erlangung der genannten stenosebezogenen Daten wird die Stenose bevorzugt durch den behandelnden Arzt analysiert und möglichst genau beschrieben. Die Erfassung der Daten kann z.B. durch Benutzereingabe über die Benutzerschnittstelle 60 erfolgen.

Hinsichtlich der Unterscheidung in vernarbte oder kalzifizierte Stenose kann z.B. die Messung der Stenosencompliance herangezogen werden.

Dazu kann das betroffene Gefäß des Patienten distal von der Stenose durch einen Ballon für die Compliance-Messung verschlossen werden. Als Druckänderung nutzt man dann den Pulsschlag im Gefäß und beobachtet die Durchmesseränderung oder man misst den Volumenstrom. Zusätzlich kann ein zweiter Ballon das Gefäß auch proximal abriegeln und man beaufschlagt den abgeriegelten Bereich mit einem hydraulischen Druck und beobachtet die Formänderung. Alternativ verschließt man das Gefäß distal und proximal der Stenose und pumpt ein Volumen in den abgeriegelten Bereich und misst dann den Druckanstieg. Diese Messungen sollten unterschiedliche Verläufe für vernarbte und kalzifizierte Stenosen ergeben.

Wie bereits einleitend dargelegt wird vorzugsweise gemäß einem grundlegenden Gedanken der vorliegenden Erfindung eine multivariate Analyse bzw. eine künstliche Intelligenz dazu verwendet, aus der aktuellen Situation und den historischen Daten die Eintrittswahrscheinlichkeit von Komplikationen wie Perforation, Dissektion oder (sehr) späte Stentthrombose abzuschätzen und eine situative Handlungsempfehlung für den Arzt zu erzeugen. Das Vorhersagemodell liegt dabei idealerweise auch offline und bevorzugt immer in einem durch Experten geprüften und freigegebenem Zustand vor.

Vorzugsweise wird gemäß einer Ausführungsform der Erfindung vor dem Start der Prozedur bzw. Angioplastie geprüft, ob die Erfolgsaussichten in einem gewünschten Bereich liegen, so dass der prozedurale Erfolg nach Möglichkeit garantiert ist.

In einer einfachsten Ausführungsform des erfindungsgemäßen Systems 1 bzw. Verfahrens ist die Funktion des Systems 1 nur analysierend und beratend. Der Arzt führt alle Behandlungsschritte weiterhin selbst durch.

In einer bevorzugten Variante des Systems ist gemäß Figur 1 vorgesehen, dass die Datenverarbeitungseinheit 6 vor Ort eine digitale Inflationspumpe 8 steuert oder regelt, die die Angioplastie nach Positionieren des Ballons 4 am richtigen Ort durchführt und laufend die Haupteinflussgröße, den besagten Aufweitdurchmesser D als Ballonparameter direkt oder indirekt überwacht. Zur Überwachung der Angioplastie ist der besagte Aufweitdurchmesser D der wichtigste Parameter beim Stentsetzen oder Nachdilatieren.

Liegt der Aufweitdurchmesser D als Messgröße am Ballon 4 nicht vor, kann die Prozedur mit etwas geringerer Präzision durch den Verlauf des Hydraulikdrucks plus des Aufweitvolumens überwacht werden. Sind bei vorgegebenem Druck das theoretische Aufweitvolumen des Ballones (Messung in vitro und dokumentiert in Compliance Data Chart) und das in-vivo gemessene Aufweitvolumen weitgehend identisch, ist die Präzision der Aufweitung hoch und das Ergebnis der Angioplastie ausreichend. Der Hydraulikdruck korreliert unabhängig vom Aufweitvolumen mit dem maximal möglichen Ballondurchmesser und damit dem Risko der Dissektion (Gefäßruptur) bei plötzlichem Brechen der Stenose.

Der Verlauf der Angioplastie wird vorzugsweise per Röntgen überwacht.

Als Ergebnis der Angioplastie erfasst die Datenverarbeitungseinheit 6 insbesondere akute Komplikationen, die sich in der Prozedur ergeben, wie z.B. eine Perforation der Gefäßwand durch Ruptur und Schweregrad der entsprechenden Blutung.

Die Güte der Vorhersage bzw. der dem Arzt durch das System gegebenen Vorschläge, insbesondere hinsichtlich des einzustellenden Sollwerts des Aufweitdurchmessers D erhöht sich mit der Qualität der Daten, deswegen sind gute Sensoren, die den Verlauf der Angioplastieprozedur beschreiben und beobachten, besonders bevorzugt.

Als spätes Ergebnis der Prozedur wird insbesondere durch die Datenverarbeitungseinheit erfasst, ob sich später Komplikationen ergeben. Besonders wichtig für die Langzeitverbesserung der klinischen Ergebnisse ist die Rückmeldung späterer Probleme wie der späten Stentthrombose.

Alle Ergebnisse bzw. Daten werden über die Datenverarbeitungseinheit 6 an die Datenbankeinheit zurückgemeldet und dort analysiert, um zu prüfen ob das multivariate Modell die Zukunft genügend genau vorhersagt.

Ein besonderer Vorteil des erfindungsgemäßen Systems 1 liegt in der fortlaufenden Verbesserung der Angioplastie durch die Auswertung einer stetig wachsenden Datenbank 70.

In Abhängigkeit von den Eingangsgrößen z.B. gesunder Gefäßdurchmesser, Stenosendurchmesser und -länge können dem Behandelnden insbesondere die statistischen Konsequenzen mit Vertrauensintervall für den maximalen Aufweitdurchdurchmesser D aufgezeigt werden.

Der zum Aufweiten des Stents benutzte Ballonkatheter 3 hilft dem Anwender somit beim Ansteuern der empfohlenen Überdehnung des Stents (um 10-20%) im Vergleich zum gesunden Gefäßdurchmesser. Dazu wird vorzugsweise die Dehnung des Ballons 4 ab dem Moment, in dem der Ballon 4 und ein gesunder sich an die Stenose anschließender Gefäßabschnitt sich stetig fortsetzen auf "0%" gesetzt und dann die fortschreitende Überdehnung des Gefäßes bzw. Ballons 4 angezeigt. Beim Erreichen des optimalen Durchmessers Dₛₒₗₗ (Sollwert des Durchmessers D) erhält der Behandler ein Signal.

Wie weiterhin in der Fig. 1 gezeigt ist, weist der Ballonkatheter 3 im zylindrischen Bereich des Ballons 4 mindestens einen Dehnungssensor 5 auf. Beide Bauteile sind insbesondere für eine Compliance von bis zu mindestens 15% idealerweise >30% und einer Auflösung von +/-5% (besser +/-1%) ausgelegt, ohne zu delaminieren oder einen Leitungsbruch zu riskieren.

Der Sensor 5 wird z.B. über eine Stromleitung mit Energie versorgt. Die Energie kann von einer Stromquelle außerhalb des Körpers des Patienten stammen, kann aber genauso gut durch eine geeignete Batterie oder Antenne im Körper des Patienten bereitgestellt werden.

Die Sensordaten S werden z.B. über eine Datenleitung oder kabellos (z.B. per Funk oder Ultraschall) nach außen zur Datenverarbeitungseinheit 6 übermittelt.

Die Sensordaten werden vorzugsweise in der Datenverarbeitungseinrichtung 6 durch einen Algorithmus verarbeitet. Der Prozessor dafür kann sich in oder außerhalb des Körpers befinden.

Gemäß einer Ausführungsform der Erfindung berechnet der Algorithmus fortlaufend aus den Mess- bzw. Sensordaten die Überdehnung des gesunden Gefäßes bzw. des Ballons 4 und überprüft ständig das Erreichen des Zielbereiches der Überdehnung (Sollwert Dₛₒₗₗ des Durchmessers D) und kündigt dieses vorzugsweise akustisch oder visuell an. Bei Überschreiten des empfohlenen Überdehnungsbereiches gibt es bevorzugt ein weiteres Signal, das dem Anwender durch seine Frequenz die Zunahme des Rupturrisikos vermittelt.

Die Daten W der Prozedur werden mit der Klassifizierung der Stenose aufgezeichnet und der prozedurale Erfolg wird dokumentiert und an die zentrale Datenbank 70 der Datenbankeinheit 7 übermittelt.

Hier ist ein Versicherungsmodell möglich, wo über eine Erfolgsgarantie des Herstellers der Klinik z.B. die Kosten des Wiedereingriffs an der gleichen Stelle erstattet werden könnte. Für dieses "Versicherungsmodell" ist die Rückmeldung der Daten zum einen Voraussetzung zum anderen sorgt die in Aussicht gestellte Erstattung für einen Anreiz, umden nötigen Datenrücklauf und damit verknüpfte Verbesserung der Überdilatationsempfehlung sicherzustellen.

Diese Daten W eignen sich weiterhin dazu, eine Korrelation zwischen Überdehnungsbereich (Sollwert Dₛₒₗₗ des Durchmessers D) und klinischem Erfolg zu ermitteln und verbesserte Handlungsempfehlungen an den Algorithmus zurückzugeben. In der Folge sind die Handlungsempfehlungen V, die automatisch insbesondere hinsichtlich des einzustellenden Sollwerts Dₛₒₗₗ Aufweitdurchmessers von der Datenverarbeitungseinheit 7 erzeugt werden, entsprechend verbessert.

Der im Rahmen der vorliegenden Erfindung bei der Angioplastie verwendete Ballon 4 kann prinzipiell aus allen thermoplastischen Ballonmaterialien hergestellt werden, die eine genügend hohe Druckfestigkeit bei geringer Wandstärke bieten. Das sind bevorzugt Polymere aus der Polyamidfamilie oder auch thermoplastische Polyurethane. Der Ballonformprozess ist vorzugsweise so ausgelegt, dass sich ein semi-compliant Ballonverhalten ergibt, das genügend Flexibilität im Zieldurchmesser in der Anwendung bietet.

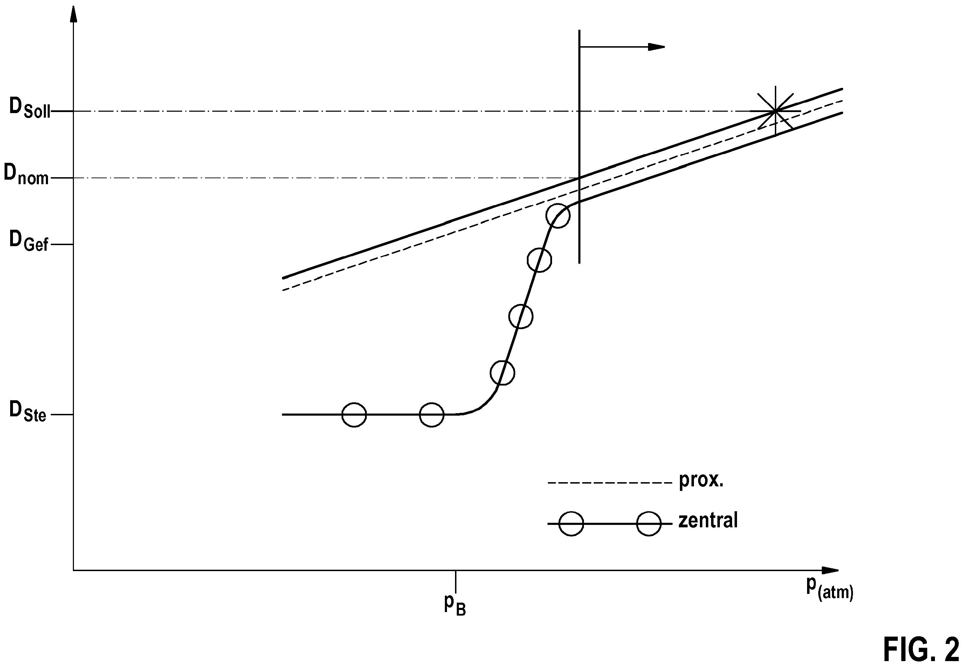

Gemäß einer vergleichsweise einfachen Ausführungsform des erfindungsgemäßen medizinischen Systems 1 kann gemäß Fig. 2 vorgesehen sein, dass lediglich der hydraulische Druck p z.B. am proximalen Kathetereingang gemessen (prox) und ein Erwartungsbereich für den Aufweitdurchmesser D angegeben wird. Idealerweise ist der Ballon 4 dabei so stark ausgelegt, dass sein Nominaldurchmesser Dₙₒₘ erst nach Brechen der Stenose beim Druck p_{B} überschritten wird. Dieser Öffhungsdruck liegt für koronare Arterien bei ca. 14 bar. Vor dem Brechen der Stenose wird der tatsächliche Durchmesser im Bereich der Stenose kleiner sein, als der auf der Compliance Data angegebene. Fig. 2 zeigt im Einzelnen den in der Stenose (zentral) vorliegenden Druck p, wobei sich der anfängliche Durchmesser der Stenose D_{Ste} durch Dehnen des Ballons 4 stetig erhöht. Der Nominaldurchmesser Dₙₒₘ des Ballons 4 liegt oberhalb des gesunden Gefäßdurchmessers D_{Gef}. Der gewünschte Durchmesser Dₛₒₗₗ des Ballons 4 entspricht der gewünschten Überdehnung des Gefäßes bzw. Ballons 4 um 10% bis 20% des gesunden Gefäßdurchmessers D_{Gef}.

Eine alternative Variante des erfindungsgemäßen medizinischen Systems 1 vergleicht das in-situ gemessene und damit erwartete Ballonvolumen mit dem realen in-vivo gemessenen Volumenstrom für jeden Balloninnendruck. Sind diese Volumina identisch, wird auch der Durchmesser in-vivo auf der gesamten zylindrischen Länge des Ballons 4 mit dem Durchmesser D in-vitro überein-stimmen.

Die genaueste Durchmessermessung erfolgt im Rahmen der vorliegenden Erfindung bevorzugt durch zumindest einen Sensor 5 (vgl. Fig. 1) im Bereich des Ballons 4. Diese Messung kann auf verschiedene Weise mit den oben beschriebenen physikalischen Prinzipien ermittelt werden.

Die Vorteile der erfindungsgemäßen Lösung liegen insbesondere in der Optimierung der Stentimplantation und Vermeidung von Stentablösung (sogenannte Wall-Apposition, wo der Stent zumindest teilweise den Kontakt zur Gefäßwand verliert) durch optimales Überdehnen von Stent und Gefäß sowie in der besseren Endothelialisierung des Stents, da ein vorgespannter Stent auch bei hohem Blutdruck nicht mehr den Kontakt zur Gefäßwandung verliert. Mit besonderem Vorteil wird dem Behandelnden durch die Warnhilfe beim Überdehnen des Ballons die Angst vor einer drohenden Dissektion genommen.

Die Konfiguration des erfindungsgemäßen Systems 1 ist weiterhin mit Vorteil so angelegt, dass das System 1 aus Fehlern bei der Angioplastie lernt. So werden der prozedurale und der klinische Erfolg durch Erlernen der optimalen Stentüberdehnung wesentlich verbessert

Insbesondere können Ablesefehler von der vormals üblichen Compliance-Data-Card vermieden werden, ein kompliziertes Umrechnen kann entfallen.

Das erfindungsgemäße System 1stellt insbesondere einen geschlossenen Feedbackkreislauf für Behandelnde und den Hersteller von Kathetern für die Angioplastie zur Verfügung. Dabei ist die Ballondurchmesseränderung als Anzeige für die Wirkung der Prozedur wesentlich geeigneter als die Änderung des Balloninnendrucks, die ein trügerisches Gefühl von Wirksamkeit vermitteln kann.

## Patentansprüche

1. Medizinisches System (1) zur datengestützten Optimierung der Einbettung eines vaskulären Implantats, insbesondere eines Stents (2), in ein Gefäß eines Patienten im Bereich einer Stenose während einer Angioplastie, mit:
- einem Ballonkatheter (3), wobei der Ballonkatheter (3) einen in einer axialen Richtung (A) erstreckten Ballon (4) aufweist und optional ein darauf angeordnetes vaskuläres Implantat, insbesondere einen Stent (2), wobei der Ballon (4) zum Aufweiten des Implantats, insbesondere des Stents (2), in radialer Richtung (R) dehnbar ist, und wobei das medizinische System (1) zumindest einen Sensor (5) aufweist, der dazu ausgebildet ist, einen Istwert eines Ballonparameters (D) zu messen,
- einer Datenverarbeitungseinheit (6), die dazu ausgebildet ist, folgende Daten (W) zu erfassen: Patientendaten, Daten zur Angioplastie, Daten zur aktuellen Situation des Patienten, Stenosedaten, einen Ballonparameter,
- einer Datenbankeinheit (7), wobei die Datenverarbeitungseinheit (6) dazu ausgebildet ist, die Daten an die Datenbankeinheit (7) zu übermitteln, wobei die Datenbankeinheit (7) dazu ausgebildet ist, die Daten in einer Datenbank (70) zu Speichern und zu analysieren, und wobei
- die Datenbankeinheit (7) dazu ausgebildet ist, eine multivariate Analyse der besagten Daten durchzuführen, um eine Eintrittswahrscheinlichkeit einer Komplikation bei der Angioplastie abzuschätzen und/oder eine Handlungsempfehlung (V) für eine die Angioplastie durchführende Person zu erzeugen und der Person über eine Benutzerschnittstelle (9) des medizinischen Systems (1) anzuzeigen.

2. Medizinisches System nach Anspruch 1, **wobei** es sich bei dem Ballonparameter um einen Durchmesser (D) des Ballons (4) in der radialen Richtung (R) des Ballons (4) handelt.

3. Medizinisches System nach Anspruch 1 oder 2, **wobei** die Handlungsempfehlung einen Vorschlag für die Auswahl eines Sollwerts des Ballonparameters (Dₛₒₗₗ) darstellt, der bei der Angioplastie einzustellen ist.

4. Medizinisches System nach einem der vorhergehenden Ansprüchen, **wobei** das medizinische System (1), insbesondere die Datenverarbeitungseinheit (6), dazu konfiguriert ist, ein Ergebnis der Angioplastie zu erfassen und an die Datenbankeinheit (7) zu übersenden, wobei die Datenbankeinheit (7) dazu konfiguriert ist, das Ergebnis in der Datenbank (70) zu speichern und bei einer multivariaten Analyse für eine weitere Angioplastie zu verwenden.

5. Medizinisches System nach einem der vorhergehenden Ansprüche, **wobei** die Patientendaten, eine oder mehrere der folgenden Informationen enthalten: das Alter des Patienten; eine Auflistung von Krankheiten des Patienten, eine Beschreibung eines momentan vorliegenden Krankheitsbildes des Patienten, eine Auflistung eines oder mehrere Medikamente, die der Patient eingenommen hat, eine Auflistung von Kontraindikationen.

6. Medizinisches System nach einem der vorhergehenden Ansprüche, **wobei** die Daten zur Angioplastie eine oder mehrere der folgenden Informationen enthalten: eine Auflistung zuvor am Patienten durchgeführter Behandlungsschritte; eine Auflistung von für den Patienten vorgesehenen Behandlungsschritten.

7. Medizinisches System nach einem der vorhergehenden Ansprüche, **wobei** die Daten zur aktuellen Situation des Patienten, eine oder mehrere der folgenden Informationen enthalten: einen Blutdruck des Patienten; einen Puls des Patienten; eine Auflistung aktuell eingenommener Medikamente und deren Dosierung.

8. Medizinisches System nach einem der vorhergehenden Ansprüche, **wobei** die Stenosedaten eine oder mehrere der folgenden Informationen enthalten: einen Ort der Stenose im Gefäß; eine Länge der Stenose, einen Durchmesser (D_{Ste}) der Stenose, einen Durchmesser (D_{Gef}) eines gesunden Abschnitts des Gefäßes, der an die Stenose angrenzt; eine Klassifizierung der Stenose.

9. Medizinisches System nach einem der vorhergehenden Ansprüche, **wobei** die Datenverarbeitungseinheit (6) dazu ausgebildet ist, einen für die Angioplastie ausgewählten Sollwert (Dₛₒₗₗ) des Ballonparameters, der bei der Angioplastie einzustellen ist, zu erfassen und an die Datenbankeinheit (7) zu übermitteln.

10. Medizinisches System nach Anspruch 3 oder 9, **wobei** die Datenbankeinheit (7) dazu konfiguriert ist, vor der Angioplastie zu prüfen, ob die besagte Eintrittswahrscheinlichkeit einer Komplikation für den Sollwert (Dₛₒₗₗ) des Ballonparameters unterhalb eines vordefinierten Schwellenwertes liegt.

11. Medizinisches System nach einem der Ansprüche 3, 9 oder 10, **wobei** das System (1) eine Inflationspumpe (8) zum Befüllen des Ballons (4) mit einem fluiden Medium (M) aufweist, wobei das System (1) dazu ausgebildet ist, einen mittels der Inflationspumpe (8) erzeugten Druck (p) des Mediums (M) im Ballon (4) automatisch so zu regeln, dass ein Istwert des Ballonparameters (D) den Sollwert (Dₛₒₗₗ) des Ballonparameters erreicht.

12. Medizinisches System nach einem der Ansprüche, **wobei** das System (1) dazu konfiguriert ist, eine Dehnung des Ballons (4) über einen gesunden Gefäßdurchmesser (D_{Gef}) des Patienten hinaus zu ermitteln und über eine Benutzerschnittstelle (60) des Systems (1) anzuzeigen, wobei der gesunde Gefäßdurchmesser (D_{Gef}) ein Durchmesser eines sich an die Stenose anschließenden gesunden Abschnitts des Gefäßes ist.

13. Medizinisches System nach den Ansprüchen 2 und 12, **wobei** das System (1) dazu ausgebildet ist, die Dehnung des Ballons (4) ab dem Moment, in dem der Istwert des Ballonparameters (D) und der gesunde Gefäßdurchmesser (D_{Gef}) zusammenfallen, auf Null zu setzen und die dann fortschreitende Dehnung des Ballons (4) über den gesunden Gefäßdurchmesser (D_{Gef}) hinaus als Prozentwert des gesunden Gefäßdurchmessers (D_{Gef}) über die Benutzerschnittstelle (60) anzuzeigen.

14. Medizinisches System nach einem der Ansprüche 3, 9 bis 11, **wobei** das System (1) dazu ausgebildet ist, ein visuelles Signal und/oder ein akustisches Signal über eine Benutzerschnittstelle (60) auszugeben, wenn der Istwert des Ballonparameters (D) den Sollwert (Dₛₒₗₗ) des Ballonparameters erreicht; und/oder wobei das System (1) dazu ausgebildet ist, ein visuelles Warnsignal und/oder ein akustisches Warnsignal über eine Benutzerschnittstelle (60) auszugeben, wenn der Istwert des Ballonparameters (D) den Sollwert (Dₛₒₗₗ) des Ballonparameters überschreitet.

15. Medizinisches System nach einem der Ansprüche 3, 9 bis 11, 14, **wobei** das System (1), insbesondere die Datenbankeinheit (7), dazu ausgebildet ist, für den Sollwert (Dₛₒₗₗ) des Ballonparameters die besagte Eintrittswahrscheinlichkeit einer Komplikation für den Patienten in Abhängigkeit einer oder mehrerer der folgenden Parameter abzuschätzen: des gesunden Gefäßdurchmessers (D_{Gef}), des Stenosendurchmessers (D_{Ste}), dem Grad der Kalzifizierung und Asymmetrie der Stenose, der Stenosenlänge.

16. Medizinisches System nach einem der Ansprüche 3, 9 bis 11, 14, 15, **wobei** das System (1), insbesondere die Datenbankeinheit (7), dazu ausgebildet ist, eine Korrelation zwischen dem Sollwert (Dₛₒₗₗ) des Ballonparameters und dem Ergebnis der Angioplastie zu ermitteln, wobei die Datenbankeinheit (7) dazu ausgebildet ist, die Korrelation zur Anpassung der besagten Handlungsempfehlung (V) zu verwenden.
